# EUROPÄISCHE PATENTANMELDUNG

(11) **EP 1 249 499 A1**
(43) Veröffentlichungstag der Anmeldung: **16.10.2002**
(21) Anmeldenummer: 01108919.0
(22) Anmeldetag: 10.04.2001
(51) Int. Cl.: C12Q 1/68, B01J 19/00, G01N 33/53

(54) **Verfahren und Vorrichtung zur Bestimmung und Selektion von Molekül-Molekül-Wechselwirkungen**

(71) Anmelder: Fraunhofer-Gesellschaft zur Förderung der angewandten Forschung e.V., 80636 München (DE)
(72) Erfinder: Gillner, Arnold, 52159 Roetgen (DE); Bremus-Köbberling, Elke, 52070 Aachen (DE); Barth, Stefan, 52064 Aachen (DE); Fischer, Rainer, 52156 Monschau (DE)
(74) Vertreter: Helbing, Jörg, Dr. Dipl.-Chem.

(57) **Zusammenfassung**

Die vorliegende Erfindung betrifft Biochips zur Dokumentation spezifischer Molekül-Molekül-Wechselwirkungen bzw. Zell-Moleküi-Wechseiwirkung, insbesondere von unbekannten, singulären Protein-Interaktionen und entsprechende Verfahren zur Selektion und Charakterisierung von Molekülen, insbesondere Polypeptiden und ihren komplementären Bindungsmolekülen.

## Beschreibung

Die vorliegende Erfindung betrifft Biochips zur Dokumentation spezifischer Molekül-Molekül-Wechselwirkungen bzw. Zell-Molekül-Wechselwirkung, insbesondere von unbekannten, singulären Protein-Interaktionen und entsprechende Verfahren zur Selektion und Charakterisierung von Molekülen, insbesondere Polypeptiden und ihren komplementären Bindungsmolekülen.

### Hintergrund der Erfindung

Die Wechselwirkungen zwischen Proteinen können derzeit durch verschiedene Techniken dargestellt werden. In neuester Zeit sind dabei Techniken entwickelt worden, die eine Selektion von Proteinbibliotheken auf immobilisierten Bibliotheken ermöglichen. Vor allem durch Kopplung von zu exprimierender DNA/RNA an das von ihr exprimierte Protein in Bakteriophagen ("Phage Display") oder durch "Ribosome Display" bzw. Profusion-Technologie, steht die genetische Information der angereicherten Proteine unmittelbar zur Verfügung. Die derzeitigen Screeningverfahren zeichnen sich dadurch aus, dass die Informationen zu den einzelnen Mitgliedern der immobilisierten Bibliotheken bekannt sind und an vordefinierten Orten auf den Chips lokalisiert sind.

Üblicherweise kommt es im Rahmen der gängigen Systeme zu einer Anreicherung eines oder mehrerer Klone aus der löslichen Proteinbibliothek in höheren Kopienzahlen. Singuläre unbekannte Klone (im Sinne eines einzigen Moleküls) können derzeit nicht unmittelbar untersucht werden; selektionierte Bakteriophagen müssen üblicherweise vermehrt werden, bevor eine detaillierte Analyse erfolgen kann. Würden unbekannte Mitglieder einer Proteinbibliothek immobilisiert, wäre eine unmittelbare Analyse des immobilisierten, durch Proteininteraktion detektierten Bindungsmoleküls derzeit ebenfalls nicht möglich.

Diese Probleme werden durch die hier eingesetzten Biochips gelöst und erlauben dadurch einen Einsatz von Verfahren zur Hochdurchsatzanalyse von Molekül-Molekül-Wechselwirkungen insbesondere von Proteinen auf Einzelmolekülebene.

### Kurzbeschreibung der Erfindung

Die vorliegende Erfindung betrifft somit
(1) ein Verfahren zur Selektion unbekannter Moleküle, umfassend die folgenden Schritte:
   (a) Immobilisieren von Molekülen einer kombinatorischen Molekülbibliothek auf einem Träger,
   (b) Anlagern von komplementären, singulären Bindungsmolekülen oder Zellen an ein bestimmtes immobilisiertes Molekül,
   (c) selektives Ablösen der miteinander interagierenden, singulären Moleküle von dem Träger und
   (d) Analysieren des in Schritt (c) abgelösten Materials;
(2) einen Biochip zur Selektion unbekannter Einzelmoleküle, umfassend einen Träger wie in (1) definiert, auf dem eine kombinatorische Molekülbibliothek immobilisiert werden kann oder eine solche immobilisiert ist, wobei gewährleistet ist, dass pro Kopplungsareal auf den Träger genau ein Molekül aufgenommen werden kann oder aufgenommen ist;
(3) ein Verfahren zur Herstellung eines Biochips, wie in (2) definiert, umfassend
   (i) Erzeugung von Kopplungsarealen auf einem Träger, die geeignet sind, genau ein Molekül aufzunehmen, und/oder
   (ii) Immobilisieren von Molekülen einer kombinatorischen Molekülbibliothek auf einem Träger; und
(4) eine Vorrichtung zur automatischen Messung der Molekül- oder Zellwechselwirkungen und deren koordinativen Zuordnung mit einer Ortsauflösung <100 nm, insbesondere zur Verwendung in einem Verfahren, wie in (1) definiert und/oder zum Auslesen eines Biochips, wie in (2) definiert.

### Figurenbeschreibung

Figur 1 zeigt den generellen Aufbau eines Mikrochips der Erfindung.
Figur 2 zeigt wie die Position des angelagerten Moleküls bzw. einer angelagerten Zelle bestimmt werden kann.
Figur 3 zeigt den schematischen Aufbau des Partikels A des Beispielsystems der Erfindung.
Figur 4 zeigt den schematischen Aufbau des Partikels B des Beispielsystems der Erfindung.
Figur 5 zeigt den schematischen Aufbau der interagierenden Partikel A & B.

### Detaillierte Beschreibung der Erfindung

"Träger" im Sinne der vorliegenden Erfindung umfasst Trägerstrukturen mit unterschiedlichen Dimensionen einschließlich Mikrostrukturen (Chips), aber auch größere Strukturen wie Objektträger und Platten im Dezimalbereich. Geeignete Trägermaterialien sind signaltransmittive Materialien, insbesondere für Licht, Wärme und/oder magnetische Felder transmittive Materialien, und besonders bevorzugt lichttransmittive Materialien (d. h. Träger aus Glas, Silikon, lichtdurchlässigem PVC usw.).

Die erfindungsgemäßen Träger (nachfolgend auch kurz "Biochips") setzen sich zusammen aus Arrays vereinzelter Moleküle aus kombinatorischen Bibliotheken (z. B. Proteine, Peptide) in einem Abstand, der gewährleistet, dass die Einzelmöleküle identifiziert werden können. Dieser Abstand ist daher u. a. abhängig von der Größe der Moleküle und liegt vorzugsweise im Bereich von 10 nm bis 100 µm, vorzugsweise 100 nm bis 5,0 µm und besonders bevorzugt 100 nm bis 1.500 nm. Dies wird ermöglicht
a) durch Kopplung einer licht- (magnetisch- oder wärme-)reaktiven Ankergruppe (z. B. o-Nitrobenzylester, Pivaloyl- oder Triazenverbindung) für Moleküle (z. B. Polypeptide) auf den Chip
b) zur Vorbereitung der Molekülkopplung, der Einstellung von kopplungsreaktiven quadratischen (jedoch auch rechtwinkligen, runden etc.) Arealen (vorzugsweise kleiner 50 nm x 50 nm, besonders bevorzugt 5 x 5 nm bis 20 x 20 nm bzw. entsprechende Areale mit anderer Geometrie) mit einem Abstand zwischen den Arealen, wie vorstehend erwähnt.
c) durch Kopplung jeweils eines Einzelmoleküls an die kopplungsreaktiven Areale.

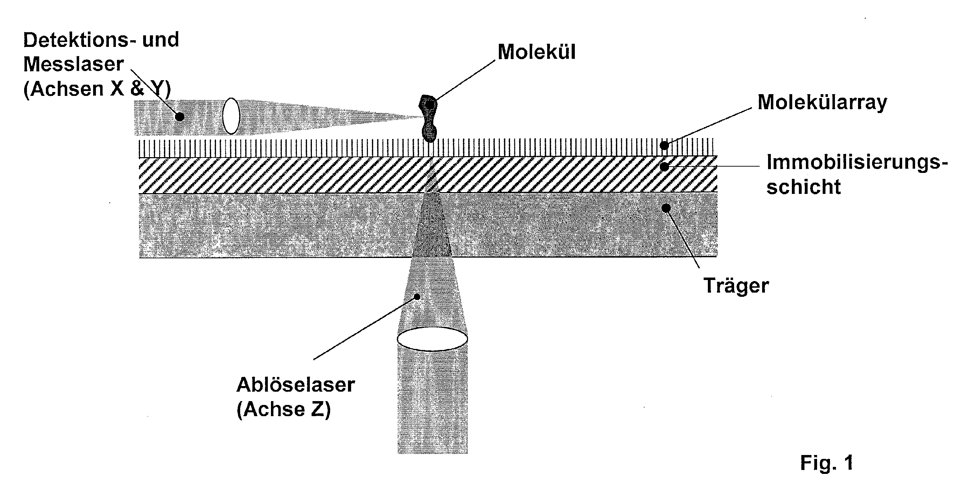

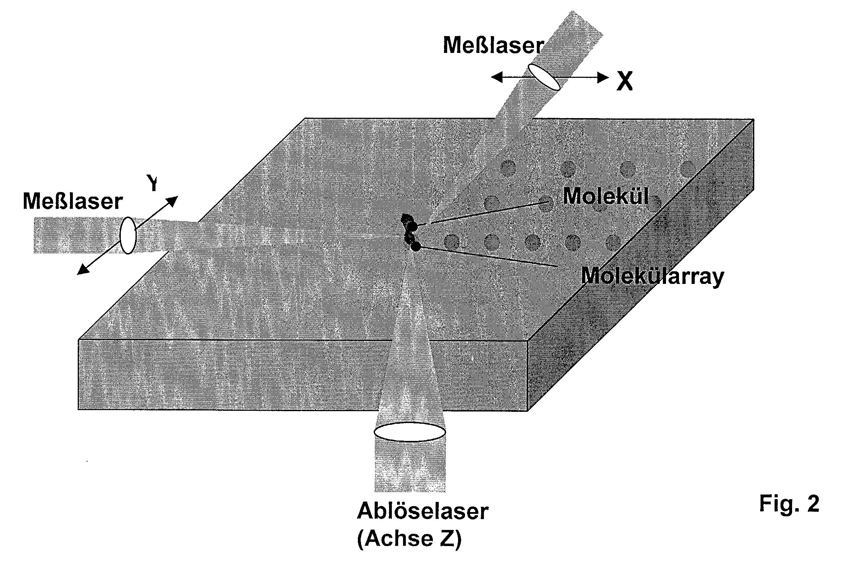

Ein erfindungsgemäßes Verfahren zur Selektion interagierender Moleküle (Zwei- und Mehrkomponentensysteme) umfasst folgende Schritte:
a) Erstellung einer ersten kombinatorischen Bibliothek insbesondere einer cDNA-Expressionsbibliothek für Bindungsproteine (beispielsweise Antikörper), alternativ einer cDNA-Expressionsbibliothek einer Zelle, oder einer synthetischen Peptidoder Molekülbibliothek, gegebenenfalls durch direkte Synthese auf den Chips,
b) Kopplung der Bibliothek über eine licht- (wärme-, oder magnetisch)-aktivierbare Ankergruppe (z. B. die nachfolgend näher definierten photoaktiven o-Nitrobenzylester, Pivaloyllinker oder Triazenverbindung) an die erfindungsgemäßen Biochips,
c) Erstellung einer zweiten kombinatorischen Bibliothek, insbesondere einer cDNA-Expressionsbibliothek einer Zelle, alternativ einer cDNA-Expressionsbibliothek für Bindungsproteine, oder einer synthetischen Peptid- oder Molekülbibliothek,
d) Screening einzelner Interaktionen zwischen der immobilisierten Bibliothek und der löslichen Bibliothek durch "Zwei(oder mehr)-Koordinaten"-Lasermessechnik (über die Achsen X und Y), oder alternative Messtechniken,
e) Gezielte, lichtabhängige Ablösung der wechselwirkenden Moleküle von dem Biochip über die dritte Achse Z (siehe auch Fig. 1 und 2),
wobei die erste Bibliothek (z. B. cDNA-Expressionsbibliothek) auf komplementäre Bindungsmoleküle nach beschriebenen Verfahren angereichert und die zweite Bibliothek z. B. insbesondere eine subtraktive cDNA-Expressionsbibliothek sein kann.

"Komplementäre Bindungsmoleküle" sind insbesondere Polypeptide, die spezifisch mit definierten Molekülen in Wechselwirkung treten. Eine spezifische Wirkung bedeutet eine Dissoziationskonstante K_{D}<10⁻⁶ mol/l, bevorzugt <10⁻⁸ mol/l.

Eine "cDNA-Expressionsbibliothek für Bindungspeptide" bezeichnet eine Vielzahl von Substanzen, bei denen in spezifischer Weise in einer Einzelkomponente die kodierende Region einer Nukleinsäure mit dem dazugehörenden exprimierten bindungsaktiven Polypeptid verknüpft ist. Typische Beispiele hierfür sind Bibliotheken komplementärer Bindungspeptide in transformierten Zellen (bevorzugt Bakterien und Hefen, besonders bevorzugt Bakteriophagen), bei denen die eingebaute Nukleinsäure funktionell translatiert wird und das Protein zytosolisch exprimiert wird, oder insbesondere als Fusionsprotein an der Oberfläche der Zelle verankert ist. Dadurch kann, ausgehend von den Eigenschaften des Bindungspeptids, eine Analyse seiner Bindungsfunktion mit dem membranassoziierten Polypeptid nach derzeitigem Stand der Technik durchgeführt und gleichzeitig die damit korrelierende Nukleinsäure eindeutig zugeordnet werden. Ähnliches gilt für das "Ribosome Display" oder Liposomendisplay oder die Profusion-Technologie.

Die Erstellung von cDNA-Expressionsbibliotheken für komplementäre Bindungspeptide in Bakteriophagen ist beispielsweise beschrieben im US Patent 5.969.108, für Display auf Ribosomen im US Patent 5.643.768.

"cDNA-Expressionsbibliotheken einer Zelle" bezeichnen eine Vielzahl unbekannter, peptidischer Einzelmoleküle, bei denen in spezifischer Weise in einer Einzelkomponente die zu einer cDNA umgewandelte kodierende Region einer mRNA mit dem dazugehörenden exprimierten Polypeptid verknüpft ist. Typisches Beispiel hierfür sind cDNA-Expressionsbibliotheken z. B. in λ-Phagen oder anderen filamentösen Bakteriophagen, die gewährleisten, dass die eingebauten Nukleinsäuren funktionell in Bakterien translatiert werden und nach Lyse der Bakterien das exprimierte Protein in entsprechenden Plaques exponiert ist, oder an der Oberfläche von Kompartimenten (Liposomen, Zellen, Bakterien, Bakteriophagen), die gewährleisten, dass die Translationsprodukte der eingebauten Nukleinsäuren funktionell an der Außenseite der Kompartimente exponiert sind. Diese cDNA-Expressionsbibliotheken beinhalten somit das Abbild der exprimierten Proteine einer Zelle, so dass ausgehend von den Eigenschaften interagierender Polypeptide durch Kombination mit dem entsprechenden komplementären Bindungspeptid durch die erfindungsgemäße "Zwei-(oder mehr) Koordinaten"-Lasertechnik Molekül-Molekül-Wechselwirkungen dokumentiert werden können.

"Synthetische Peptid- oder Molekülbibliothek" bezeichnet eine Vielzahl von Peptiden oder Molekülen, die über kombinatorische Synthese, wodurch im Idealfall alle theoretisch kombinierbaren Molekülzusammensetzungen verfügbar werden, zusammengesetzt worden ist.

"Immobilisierung" bedeutet, dass die Mitglieder der einen Bibliothek an einer festen Matrix gebunden werden (z. B. Immobilisierung mittels Ankergruppen wie o-Nitrobenzylester, Pivaloyl- oder Triazenverbindungen), die nach Dokumentation der Wechselwirkung mit dem zweiten Peptid, besonders leicht durch Licht-(Wärme-, oder Magnet-)Reaktion getrennt werden können. Geeignete Ankergruppen für die Immobilisierung sind insbesondere die folgenden in WO 95/31429 und WO 99/67619 genannten Verbindungen:
o-Nitrobenzylester-basierte Fotolinker (Linker I):
   • λ_{Spalt} = 365 nm 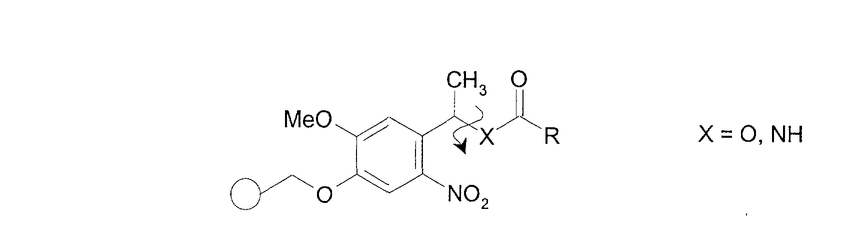
Pivaloyl-Linker (Linker II):
   • λ_{Spalt} ≈ 280 bis 340 nm 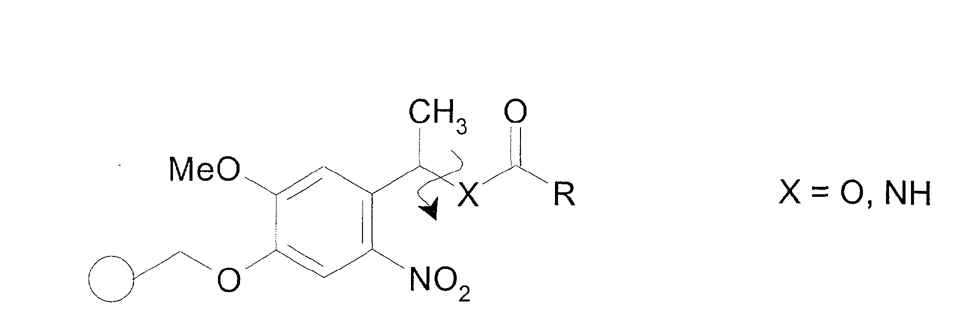
und Triazenverbindungen.

Der generelle Aufbau der Biochips ist in Fig. 1 dargestellt. Basis der Biochips bildet eine Quarz- bzw. Glasplatte, alternativ kann auch ein Kunststoffträger dienen, der jedoch signaltransmittiv (für Licht, Wärme, magnetische Felder) sein muss. Auf diesem Träger wird zunächst eine Haftschicht (z. B. aus Polylysinderivaten) und dann eine Immobilisierungsschicht aufgetragen, die einerseits an dem Träger haftet und andererseits, auf der Protein-zugewandten Seite, mit den zu immobilisierenden Einzelmolekülen aus den kombinatorischen Bibliotheken eine feste (kovalente) Bindung eingeht. Die Immobilisierungsschicht besteht entweder aus chemischen Molekülen oder Verbindungen, die durch Lichtanregung chemisch gespalten werden bzw. Molekülen oder Verbindungen, die durch thermische Einwirkung getrennt werden.

Nach dem Beladen mit Haftschicht und Fotolinkerschicht werden die kopplungsaktiven Areale, d. h., die gewünschten Strukturen auf dem Träger erzeugt. Alternativ kann auch die Fotolinkerschicht nach Einstellung der Struktur der Haftschicht aufgebracht werden.

Diese Immobilisierungsschicht wird mit Einzelmolekülen einer kombinatorischen Bibliothek so beladen, dass gewährleistet ist, dass ein Molekül an einem, nicht aber an zwei benachbarten Orten bindet. Dies wird dadurch erzielt, dass ein Abstand von 10 nm bis 1000 µm (vorzugsweise 100 nm bis 5.000 nm bzw. 100 nm bis 1.500 nm) in Abhängigkeit von der maximalen Einzelmolekülgröße zwischen den Arrays gewährt wird, d. h. die Moleküle werden nach einem festgelegten Ortsraster vereinzelt.

Die so vorbereiteten Biochips können in dieser Form einer zweiten kombinatorischen Bibliothek ausgesetzt werden. Dabei lagern sich bestimmte Moleküle aus der löslichen Bibliothek an einzelne Moleküle der immobilisierten Bibliothek des Biochips an. Mittels einer geeigneten Messtechnik wird nun die Position des angelagerten Moleküls bzw. auch einer angelagerten Zelle an dem jeweiligen Protein mit hoher Ortsauflösung bestimmt (Fig. 2). Als Messtechniken können dabei folgende Verfahren eingesetzt werden:
Fluoreszenzmessungen an zuvor mit einem Fluoreszenzmarker versehenen Molekül (Peptid, Protein, konjugiertes Ribosom, konjugierter Bakteriophage, Liposom, markierte Partikel und Zellen),
simulierte Ramanemissionen aus dem angelagerten Molekül (Peptid, Protein, konjugiertes Ribosom, konjugierter Bakteriophage, Liposom, markierte Partikel und Zellen),
magnetische Detektion des Anlagerungsortes über ein Magnetbeadgekoppeltes Molekül (Peptid, Protein, konjugiertes Ribosom, konjugierter Bakteriophage, Liposom, markierte Partikel und Zellen),
Atomic-Force-Mikroskopie, mit der eine lokale Anreicherung eines Moleküls (Peptid, Protein, konjugiertes Ribosom, konjugierter Bakteriophage, Liposom, markierte Partikel und Zellen) mit hoher Ortsauflösung bestimmt werden kann,
wobei die Meßmethode so ausgelegt sein muss, dass gegebenenfalls eine Ortsauflösung < 100 nm erzielt werden kann.

Nach Waschen des Biochips und Entfernung sämtlicher nicht angelagerter Bestandteile und Restmoleküle erfolgt die eigentliche Messung der Molekül-Interaktion am Anlagerungsort (über die Achsen X und Y). Nach Bestimmung des Anlagerungsortes wird mittels eines zweiten Scanningsystems ein Laserstrahl durch den Biochip, d.h. durch den Träger von unten (Achse Z) auf die Immobilisierungsschicht (z. B. aus der o-Nitrobenzylester, Pivaloyl- oder Triazenverbindungen, wie vorstehend definiert) gerichtet. Die Position dieser Belichtung entspricht dabei der zuvor bestimmten Position des angelagerten Moleküls. Durch kurzzeitige Bestrahlung der Immobilisierungsschicht werden anschließend die chemischen Bindungen der photolabilen Linker- oder Ankergruppe der Immobilisierungsschicht gespalten, bzw. die Immobilisierungsschicht thermisch aufgelöst. Dadurch können die an diesem Ort interagierenden Moleküle wieder spezifisch abgelöst werden. Dies bedeutet, dass sowohl das zuvor immobilisierte Molekül (Peptid, Protein, konjugierten Ribosom, konjugierten Bakteriophagen, Liposom, markierte Partikel), als auch das damit verbundene lösliche Molekül (Peptid, Protein, konjugierten Ribosom, konjugierten Bakteriophagen) für die nachfolgende Analyse zur Verfügung stehen.

Über die nachgeschaltete Analyse der so selektionierten, interagierenden Moleküle, z. B. über 'matrix associated laser desorption/ionization' (MALDI) oder 'electrospray ionization' (ESI) mit anschließender Massenanalyse im Flugzeit- (TOF oder oTOF), Quadrupol- (Q), Ionenfallen- (IT), Fourier-Tranform- (FTMS oder FT-ICR) Massenspektrometer, 'post-source decay' PSD, 'in source decay' (ISD), 'surface plasmon resonance' (SPR), Multispektralanalyse, Fluoreszenz-Korrelations-Spektroskopie (FCS), oder Polymerasekettenreaktion (PCR) läßt sich die Struktur des Proteins und seiner DNA ableiten und gegebenenfalls seine DNA vervielfältigen.

Ungeachtet der obengenannten Analyse und Ablösemechanismen können auch andere ortsauflösende Detektionsmethoden und Aktivierungstechnologien verwendet werden.

### Beispielsystem

### Schritt 1: Synthese der Mikrochips

Auf Quarz- oder Glassubstrat wird zunächst eine Haftschicht aus Polylysinderivaten, dann die Fotolinkerschicht aus Linker I (mit X = 0) ein Partikel A-reaktiver Ligand (hier: Biotin) daran gekoppelt. Durch Lasertechnik werden als Strukturierung Areale mit <50 nm Durchmesser in einem Abstand von 1.2 µm generiert. Da in dem hier vorgestellten Beispielsystem die Länge der zu immobilisierenden Partikel (Partikel A) ∼ 1 µm beträgt und verhindert werden soll, dass ein Partikel A an zwei Kopplungsareale bindet, muss dieser Mindestabstand zwischen den Kopplungsarealen gewährleistet sein.

### Schritt 2: Generierung immobilisierter Molekülbibliotheken

### 2.1 Synthese einer kombinatorischen Molekülbibliothek (am Beispiel einer scFv-Bakteriophagenbibliothek):

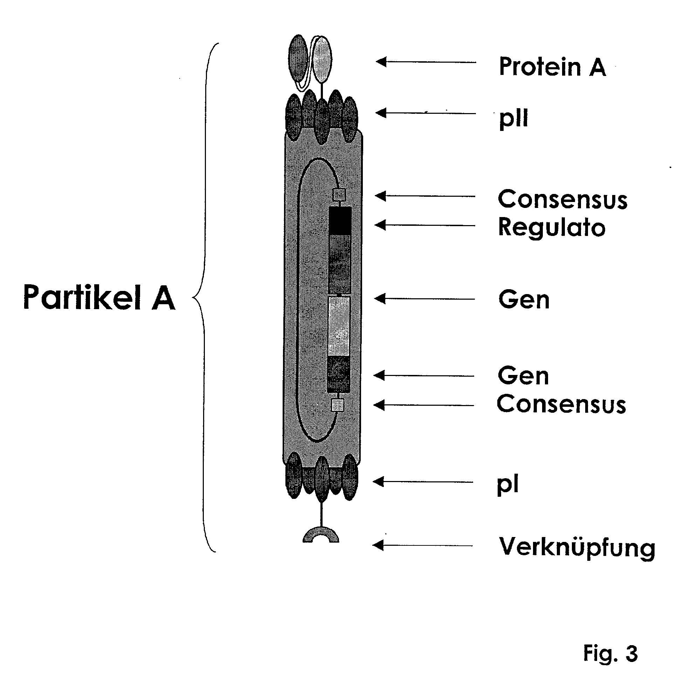

Gemäß G. E. Stoica et al., J. Biol. Chem. (2001) wird eine kombinatorische scFv-Bakteriophagenbibliothek (hier: mit Reaktivität gegen Pankreaskarzinom) zur Immobilisierung auf den Mikrochips erstellt, deren Einzelkomponenten (Partikel A) folgendermaßen charakterisiert sind (Fig. 3):

An einem Ende des Partikels befindet sich das Protein A (hier: ein unbekanntes scFv oder Fab der zu immobilisierenden Bibliothek) das kovalent und funktionell an ein Oberflächenprotein des Partikels A (hier Protein III (pIII) eines filamentösen Bakteriophagen) gekoppelt ist.

Das gegenüberliegende Ende desselben Partikels ist so modifiziert, dass eine gerichtete Kopplung des Partikels A an ein Kopplungsareal des Mikrochips durch die Aktivität der Verknüpfung A gewährleistet ist (in diesem Fall: Streptavidin oder eine modifizierte Streptavidinbindungskomponente oder ein anti-Biotin scFv).

Partikel A trägt ein Phagmid mit der genetischen Information zur Expression des Proteins A als Fusionsprotein mit einem Oberflächenprotein (hier Gen A fusioniert mit Gen III gesteuert durch den Regulator). Zusätzlich befindet sich auf dem Phagmid die Anlagerungsstellen (Consensus A1 und A2) für die spezifischen Primer A1 und A2.

### 2.2 Kopplung einer Bibliothek:

Die nach Schritt 2.1 konstruierte Bibliothek wird auf die vorbereiteten Mikrochips aufgebracht; es kommt zu einer Bindung der Einzelkomponenten an die Kopplungsareale durch die Interaktion zwischen dem Partikel A-reaktiven Liganden und der Verknüpfung A (hier: Biotin/Streptavidin oder Biotin/anti-Biotin scFv). Hierdurch wird gewährleistet, dass mit hoher Wahrscheinlichkeit an jedem Kopplungsareal ein anderer, unbekannter Partikel sitzt.

### Schritt 3: Generierung der löslichen Molekülbibliotheken (am Beispiel einer cDNA-Expressionsbibliothek aus primärem Tumormaterial)

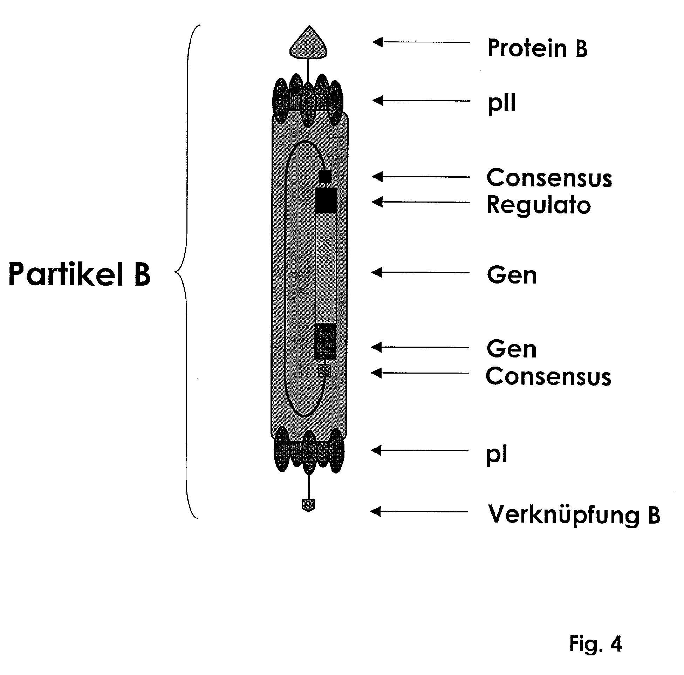

Nach X. Cai et al., Proc. Natl. Acad. Sci. USA 92(14):6531-41 (1995) wird eine lösliche cDNA-Expressionsbibliothek (hier: cDNA generiert aus der mRNA primärer Pankreaskarzinomzellen) erstellt, deren Einzelkomponenten (Partikel B) folgendermaßen charakterisiert sind (Fig. 4):

An einem Ende des Partikels befindet sich das Protein B (hier: ein unbekanntes Translationsprodukt aus den primären Pankreaskarzinomzellen) das kovalent und funktionell an ein Oberflächenprotein des Partikels B (hier Protein III (pIII) eines filamentösen Bakteriophagen) gekoppelt ist.

Das gegenüberliegende Ende desselben Partikels ist so modifiziert, dass eine gerichtete Kopplung des Partikels B über die Verknüpfung B an ein Sortierungspartikel gewährleistet ist (in diesem Fall: poly-Histidin-Markierung).

Partikel B trägt ein Phagmid mit der genetischen Information zur Expression des Proteins B als Fusionsprotein mit einem Oberflächenprotein (hier Gen B fusioniert mit Gen III gesteuert durch den Regulator). Zusätzlich befindet sich auf dem Phagmid die Anlagerungsstellen (Consensus B1 und B2) für die spezifischen Primer B1 und B2.

Optional kann Partikel B zusätzlich mit GFP-Fusionen an bspw. Oberflächenprotein VIII versehen sein, um eine Detektion über Fluoreszenztechniken zu ermöglichen.

### Schritt 4: Detektion der einzelnen Interaktionen

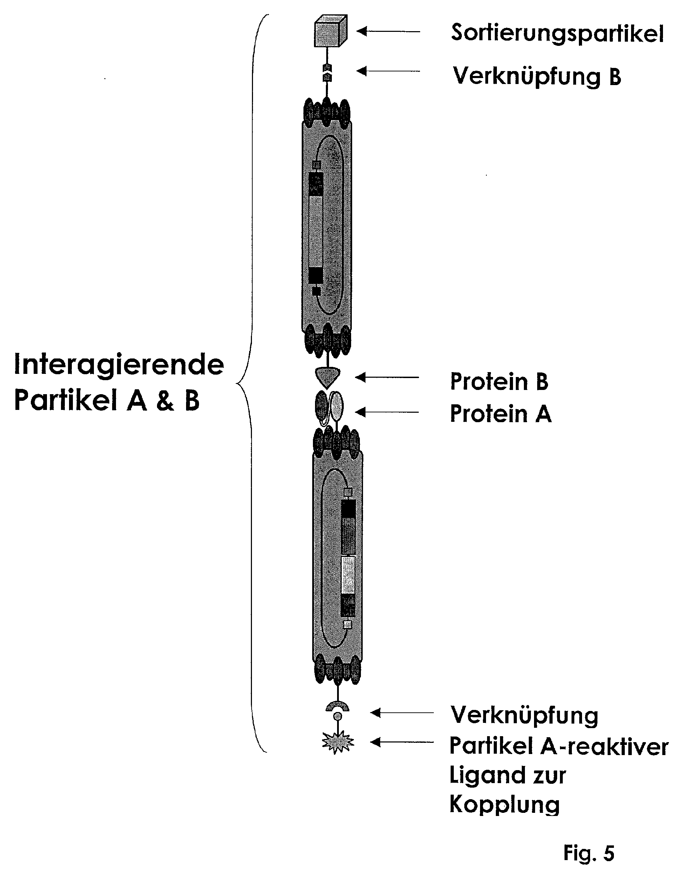

Die lösliche Proteinbibliothek wird auf die immobilisierte Proteinbibliothek gegeben, und durch mehrere Waschschritte werden unspezifische Bindungsaktivitäten zwischen den Bibliotheken eliminiert. Nach Kopplung an das Sortierungspartikel (hier: magnetisches Partikel mit Bindungsaktivität für den poly-Histidin-Tag, bspw. Ni-NTA oder "high graft") - es besteht optional die Möglichkeit ein magnetisches Feld anzulegen und so eine vektorielle Ausrichtung der interagierenden Partikel A & B zu erhalten (s. Fig. 5) - erfolgt die Messung der entsprechend-interagierenden Partikel über bspw. SNOM, oder 2-D-Scanning-Fluoreszenztechnik (Fig. 2). Hieraus ergibt sich eine Zuordnung der detektierten Interaktion zu den Koordinaten X und Y.

### Schritt 5: Spezifisches Lösen der interagierenden Moleküle

Die Koordinaten der detektierten Interaktion werden mit den Koordinaten der Kopplungsareale abgeglichen (interne Kontrolle). Der Ablöselaser (SNOM, alternativ: CW-UV-Quellen) wird auf diese Koordinaten justiert und wirkt über die Achse Z auf die auf dem Mikrochip aufgebrachte Fotolinkerschicht (Fig. 2), wodurch es zu einem gerichteten Ablösen der interagierenden Partikel kommt.

### Schritt 6: Erstes Proof-of-Principle

Nach X. Cai et al., Proc. Natl. Acad. Sci. USA 92(14):6531-41 (1995) wird eine cDNA-Miniexpressionsbibliothek bestehend aus CD30- und CEA-Antigen zur Immobilisierung auf den Mikrochips erstellt, deren Einzelkomponenten wie in Fig. 3 beschrieben charakterisiert sind. An einem Ende des Partikels befinden sich die benannten Antigene die kovalent und funktionell an das Protein III (pIII) filamentöser Bakteriophagen gekoppelt sind. Das gegenüberliegende Ende desselben Partikels ist so modifiziert, dass eine gerichtete Kopplung des Partikels A an ein Biotin-Kopplungsareal des Mikrochips durch Antibiotin-Bindungsliganden möglich ist. Die beiden verschiedenen Partikel A tragen jeweils ein Phagmid mit der genetischen Information zur Expression der verschiedenen Antigene als Fusion mit Gen III gesteuert durch den Regulator. Die Minibibliothek wird auf die vorbereiteten Mikrochips aufgebracht; es kommt zu einer Bindung der Einzelkomponenten an die Kopplungsareale durch die Interaktion zwischen Biotin/anti-Biotin Liganden. Die Verteilung der Antigentragenden Bakteriophagenpartikel an den Kopplungsarealen korreliert direkt mit den prozentualen Anteilen der Partikel in der Minibibliothek.

Nach X. Cai et al., Proc. Natl. Acad. Sci. USA 92(14):6531-41 (1995) wird eine lösliche scFv-Bakteriophagenminibibliothek bestehend aus anti-CD30, anti-CEA und anti-MUC-1 erstellt, deren Einzelkomponenten (Partikel B) folgendermaßen charakterisiert sind (Fig. 4): An jeweils einem Ende der Partikel befinden sich die scFv die kovalent und funktionell an das Protein III (pIII) der filamentösen Bakteriophagen gekoppelt ist. Das gegenüberliegende Ende derselben Partikel ist so modifiziert, daß eine gerichtete Kopplung der Partikel B über die poly-Histidin-Markierung an ein Ni²⁺-NTA-gekoppeltes Sortierungspartikel gewährleistet ist. Die Partikel B tragen jeweils ein Phagmid mit der genetischen Information zur Expression der scFv als Fusion mit Gen III gesteuert durch den Regulator.

Nach Einstellung verschiedener Verdünnungen sowohl von CD30 und CEA für die immobilisierte als auch von anti-CD30, anti-CEA und anti-Muc-1 für die lösliche Minibibliothek, werden miteinander wechselwirkende Partikel detektiert, über den Ablöselaser von dem Träger gelöst, gesammelt und über das gekoppelte magnetische Sortierungspartikel an entsprechende Säulen gebunden. Nach intensivem Waschen werden die magnetisch gebundenen Partikel eluiert und durch Polymerasekettenreaktion analysiert bzw. durch *invitro*-Bindungsstudien (ELISA, Durchflusszytometrie) der scFv-tragenden Bakteriophagen, die Bindung an CD30- und CEA-positive Zellen- und Membranfraktionen bestätigt.

### Schritt 7: Auffangen der interagierenden Moleküle

Die aneinandergekoppelten Partikel A & B werden über den Sortierungspartikel (hier: magnetisches Partikel mit Bindungsaktivität für das poly-Histidin-Tag, bspw. durch Metall-Chelate oder "high graft") durch Mikrofluidsysteme in die finalen Mikroreaktionsgefäße abgeleitet.

### Schritt 8: Analyse der interagierenden Moleküle

In den Mikroreaktionsgefäßen werden die kodierenden Regionen der sortierten Partikel A & B gleichzeitig durch Einsatz der spezifischen Primer A1/A2 und B1/B2 durch Polymerasekettenreaktion amplifiziert. Aliquots dieses Reaktionsansatzes können durch parallele Sequenzierungsansätze analysiert und dadurch gleichzeitig die Sequenzen der Gene A & B ermittelt und daraus mit Standardmethoden die kodierten Proteinsequenzen abgeleitet werden.

Die Aktivität der verfügbaren Translationsprodukte wird nach Expression in bekannten Systemen *in vitro* und *in vivo* charakterisiert.

## Patentansprüche

1. Verfahren zur Selektion unbekannter Moleküle, umfassend die folgenden Schritte:
(a) Immobilisieren von Molekülen einer kombinatorischen Molekülbibliothek auf einem Träger,
(b) Anlagern von komplementären, singulären Bindungsmolekülen oder Zellen an ein bestimmtes immobilisiertes Molekül,
(c) selektives Ablösen der miteinander interagierenden, singulären Moleküle von dem Träger und
(d) Analysieren des in Schritt (c) abgelösten Materials.

2. Verfahren nach Anspruch 1, wobei der Träger aus signaltransmittivem Material, insbesondere für Licht, Wärme und/oder magnetische Felder transmittivem Material, und besonders bevorzugt aus lichttransmittivem Material besteht.

3. Verfahren nach Anspruch 1 oder 2, wobei jeweils ein Einzelmolekül in kopplungsreaktiven Arealen, insbesondere in Arealen mit einer Abmessung von kleiner 50 nm x 50 nm und/oder einem Abstand von 10 nm bis 100 µm immobilisiert ist.

4. Verfahren nach Anspruch 3, wobei Schritt (a) das Aufbringen einer licht-, wärme- oder magnetisch-reaktiven Ankergruppe, insbesondere photolabile Linker wie o-Nitrobenzylester, Pivaloyl- oder Triazenverbindungen oder ein thermisch-empfindliches Polymer auf den Träger und das Einstellen der kopplungsreaktiven Areale umfasst.

5. Verfahren nach einem oder mehreren der Ansprüche 1 bis 4, wobei die lösliche Molekülbibliothek eine cDNA-Expressionsbibliothek für Bindungsproteine, eine cDNA-Expressionsbibliothek einer Zelle oder eine synthetische Peptid- oder Molekülbibliothek ist und insbesondere die Moleküle Peptide, Proteine, konjugierte Ribosomen oder konjugierte Bakteriophagen sind.

6. Verfahren nach einem oder mehreren der Ansprüche 1 bis 5, wobei die komplementäre Molekülbibliothek ausgewählt ist aus Peptiden, Proteinen, konjugierten Ribosomen, konjugierten Bakteriophagen, Liposomen, markierten Partikeln.

7. Verfahren nach einem oder mehreren der Ansprüche 1 bis 6, wobei nach Schritt (b) ein oder mehrere Waschschritte erfolgen.

8. Verfahren nach einem oder mehreren der Ansprüche 1 bis 7, bei dem die Analyse des Ortes der Molekül- Zell- oder Molekül-Zell-Interaktion durch ein optisches, magnetisches oder profilometrisches Messverfahren erfolgt.

9. Verfahren nach einem oder mehreren der Ansprüche 1 bis 8, wobei eine kombinatorische Bibliothek auf einem transparenten Träger immobilisiert ist und diese Immobilisierungsschicht selektiv durch photonische oder thermische Wechselwirkung aufgelöst werden kann.

10. Verfahren nach einem oder mehreren der Ansprüche 1 bis 9, wobei Schritt (d) durch MALDI ("matrix associated laser desorption/ionization") oder ESI ("electrospray ionization") mit anschließender Massenanalyse, SPR ("surface plasmon resonance"), Multispektralanalyse, Fluoreszenz-Korrelations-Spektroskopie (FCS), oder Polymerasekettenreaktion (PCR) erfolgt.

11. Biochip zur Selektion unbekannter Einzelmoleküle, umfassend einen Träger wie in Ansprüchen 1 bis 4 definiert, der zur Immobilisierung einer kombinatorischen Molekülbibliothek geeignet ist oder mit einer solchen immobilisiert ist, auf dem eine kombinatorische Molekülbibliothek immobilisiert werden kann oder eine solche immobilisiert ist, wobei gewährleistet ist, dass pro Kopplungsareal auf den Träger genau ein Molekül aufgenommen werden kann oder aufgenommen ist.

12. Verfahren zur Herstellung eines Biochips gemäß Anspruch 11, umfassend
(i) Erzeugung von Kopplungsarealen auf einem Träger, die geeignet sind, genau ein Molekül aufzunehmen, und/oder
(ii) Immobilisieren von Molekülen einer kombinatorischen Molekülbibliothek auf einem Träger.

13. Vorrichtung zur automatischen Messung der Molekül- oder Zellwechselwirkungen und deren koordinativen Zuordnung mit einer Ortsauflösung <100 nm, insbesondere zur Verwendung in einem Verfahren gemäß Ansprüchen 1 bis 10 und/oder zum Auslesen eines Biochips gemäß Anspruch 11.

14. Vorrichtung nach Anspruch 13, die zur Sortierung der interagierenden Moleküle geeignet ist.

15. Vorrichtung nach Anspruch 13, die zur Analyse der interagierenden Moleküle geeignet ist und vorzugsweise das detektierte Molekül mittels MALDI ("matrix associated laser desorption/ionization") oder ESI ("electrospray ionization"), SPR, Multispektralanalyse, FCS, oder PCR identifiziert.
